# EUROPÄISCHE PATENTSCHRIFT

(11) **EP 1 560 921 B1**
(45) Veröffentlichungstag und Bekanntmachung des Hinweises auf die Patenterteilung: **28.03.2007**
(21) Anmeldenummer: 03810436.0
(22) Anmeldetag: 05.11.2003
(51) Int. Cl.: C12P 7/62, C08F 220/06, C09D 133/08, C09D 133/10

(54) **UV-AKTIVE BINDEMITTEL**
UV-ACTIVE BINDING AGENT
LIANTS DURCISSABLES PAR UV

(30) Priorität: 05.11.2002 DE 10251729
(43) Veröffentlichungstag der Anmeldung: 10.08.2005
(73) Patentinhaber: BASF Coatings AG, 48165 Münster (DE)
(72) Erfinder: RINK, Heinz-Peter, 48153 Münster (DE); DEUTRICH, Susanne, 48165 Münster (DE); MEISENBURG, Uwe, 47051 Duisburg (DE); JOOST, Karl-Heinz, 48317 Drensteinfurt (DE); HÄRING, Dietmar, 69198 Schriesheim (DE); HAUER, Bernhard, 67136 Fussgönheim (DE)
(74) Vertreter: Isenbruck, Günter
(86) Internationale Anmeldenummer: PCT/EP2003/012322
(87) Internationale Veröffentlichungsnummer: WO 2004/042069

(56) Entgegenhaltungen:
- EP-A- 0 999 229
- EP-A- 0 999 230
- WO-A-01/46286
- TOR R ET AL: "ENZYMATICALLY CATALYSED TRANSESTERIFICATIONS OF ACRYL AND METHACYRL MONOMERIC ESTERS" ENZYME AND MICROBIAL TECHNOLOGY, STONEHAM, MA, US, Bd. 12, April 1990 (1990-04), Seiten 299-304, XP000910508 ISSN: 0141-0229
- PAVEL K ET AL: "ENZYMES IN POLYMER CHEMISTRY" POLYMER BULLETIN, SPRINGER VERLAG. HEIDELBERG, DE, Bd. 21, Nr. 5, 1. Mai 1989 (1989-05-01), Seiten 535-540, XP000051853 ISSN: 0170-0839 in der Anmeldung erwähnt

## Beschreibung

Die Erfindung betrifft ein Verfahren zur Herstellung strahlenhärtbarer und/oder Dual-Cure-härtbarer Poly(meth)acrylate, die Poly(meth)acrylate selbst, deren Verwendung als Komponente bei der Herstellung von Dispersionen oder als Komponente in Lackformulierungen, Lackformulierungen enthaltend die erfindungsgemäßen Poly(meth)acrylate sowie ein Verfahren zur Herstellung der Lackformulierungen und deren Verwendung.

UV- und Dual-Cure-härtbare Poly(meth)acrylate sind für den Einsatz in Deckbeschichtungen von besonderem Interesse. Poly(meth)acrylate weisen im Allgemeinen ausgezeichnete Witterungsstabilitäten im Außenbereich auf. In Verbindung mit einer UV-Härtungstechnologie lassen sich zusätzliche Vorteile erzielen. So kann zum Beispiel die Kratzfestigkeit der Beschichtung signifikant erhöht und damit die Beschichtungsperformance verbessert werden. Besonders wesentlich sind jedoch Verbesserungen in der Anwendung, insbesondere eine sehr schnelle Trocknung der Beschichtungsmaterialien. Diese Eigenschaft ist maßgeblich für schnelle Verarbeitungstechniken.

Gemäß dem Stand der Technik werden UV-härtbare Polyacrylate durch Copolymerisation von Glycidylmethacrylat und anschließender thermischer Umsetzung mit Acrylsäure in Anwesenheit eines Katalysators umgesetzt (DE-A 2 436 186, EP-A 0 650 978). Nachteilig sind bei dieser Herstellungsweise des Standes der Technik die dabei auftretenden Nebenreaktionen und Farbverschlechterungen. Aufgrund der Reaktionsbedingungen, insbesondere der hohen Temperaturen, bei denen die Umsetzung durchgeführt wird, ist der Einsatz von Stabilisatoren zur Verhinderung der radikalischen Polymerisation der eingesetzten Acrylsäure zwingend erforderlich.

Eine herkömmliche saure Veresterung von hydroxyfunktionelle Seitenketten aufweisenden Poly(meth)acrylaten mit Acrylsäue ist nicht möglich, weil dabei die Esterbindungen des Poly(meth)acrylats gespalten werden.

Aus dem Stand der Technik ist die Funktionalisierung von polymeren Verbindungen mit (Meth)acrylsäure und/oder (Meth)acrylsäureestern bekannt.

EP-A 0 999 230 und EP-A 0 999 229 betreffen Verfahren zur Herstellung von (Meth)acrylsäureestern von hydroxyfunktionellen Siloxanen und/oder polyalkylenmodifizierten Siloxanen ('230) sowie von Polyoxyalkylenen ('229) durch Ver- oder Umesterung der Siloxane bzw. der Polyoxyalkylene mit (Meth)acrylsäure und/oder (Meth)acrylsäureestern in Anwesenheit eines Enzyms. Gemäß EP-A 0 999 230 und EP-A 0 999 229 werden jedoch lediglich die genannten speziellen Polymere mit (Meth)acrylsäure und/oder (Meth)acrylsäureestern umgesetzt. Eine Umsetzung von Poly(meth)acrylaten ist nicht erwähnt.

E. Marechal et al., Polymer Bulletin 26, 55 bis 62 (1991) betrifft die Umesterung von Oligo(methacrylaten), die Ester-Endgruppen aufweisen, mit Allylalkohol in Anwesenheit von Lipase. Es erfolgt lediglich eine Umesterung an den Endgruppen.

H. Ritter et al., Polymer Bulletin 21, 535 bis 540 (1989) betrifft die Lipase-katalysierte Acetylierung von Methacrylsäurepolymeren, die OH-Gruppen enthalten. Die Acetylierung erfolgt in Anwesenheit von Vinylacetat. Durch Umsetzung mit Vinylacetat wird eine sehr gute Abgangsgruppe erhalten, wobei der entstehende Aldehyd leicht aus dem Reaktionsgemisch zu entfernen ist. Dennoch beträgt die Reaktionsdauer 2 bis 15 Tage.

H. Ritter et al., Makromol. Chem. 193, 323 bis 328 (1992) betrifft die enzymatisch katalysierte Acylierung von OH-Gruppen enthaltenden kammartigen Methacrylsäurepolymeren mit aktiven Estern, wie Vinylacetat, Phenylacetat, 4-Fluorophenylacetat und Phenylstearat. Eine Veresterung der Polymere mit Acrylaten ist nicht erwähnt. Die Reaktionszeiten der Umsetzung gemäß Ritter et al. sind sehr lang (2, 4 und 6 Tage).

Die Aufgabe der vorliegenden Erfindung ist die Bereitstellung eines schonenden und selektiven Verfahrens zur Herstellung von mit (Meth)acrylsäure bzw. (Meth)acrylaten funktionalisierten Poly(meth)acrylaten, das gegenüber dem bekannten Herstellungsverfahren ausgehend von Glycidylmethacrylat von preiswerteren Ausgangssubstanzen ausgehen kann und variabler ist und eine schonendere Herstellung ermöglicht, so dass neuartige mit (Meth)acrylgruppen substituierte Poly(meth)acrylate zugänglich sind.

Die Aufgabe wird durch ein Verfahren zur Herstellung UV- und/oder Dual-Cure-härtbarer Poly(meth)acrylate gelöst, umfassend die folgenden Schritte
a) Herstellung eines hydroxyfunktionelle Seitenketten aufweisenden Poly(meth)acrylats durch Polymerisation von
   aa) mindestens einem (Meth)acrylat der allgemeinen Formel (1), als Komponente A 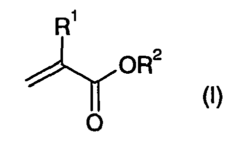 worin
      - R¹: H, CH₃ oder CH₂OH bedeutet, und
      - R²: ein Alkyl- oder Cycloalkylrest ist, der gegebenenfalls mit funktionellen Gruppen wie Acryl-, Ether-, Amino-, Epoxy-, Halogen- oder Sulfonsäuregruppen substituiert ist, bevorzugt ein C₁- bis C₁₈-Alkylrest, besonders bevorzugt ein C₁- bis C₈-Alkylrest, ganz besonders bevorzugt ein nicht mit funktionellen Gruppen substituierter C₁- bis C₈-Alkylrest, insbesondere ein Methl-, Ethyl-, n-Propyl-, Iso-Propyl-, n-Butyl-, Iso-Butyl-, 2-Ethylhexyl-, tert.-Butyl-, Cyclohexyl-, tert.-Butylcyclohexyl-, Isobornyl- oder Trimethylcyclohexylrest; und
   ab) mindestens einem Hydroxyalkyl(meth)acrylat der allgemeinen Formel (II), als Komponente B 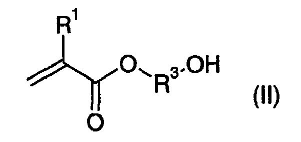 worin
      - R¹: H, CH₃ oder CH₂OH bedeutet, und
      - R³: -(CH₂)ₙ-, -CH₂-CH(CH₃)-CH₂- oder -CH₂CH(CH₃)- oder -CH(CH₃)CH₂- oder 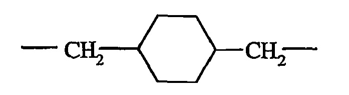
      bedeutet,
      - n: mindestens 2, bevorzugt 2 bis 8, besonders bevorzugt 2 bis 6, ganz besonders bevorzugt 2 bis 4 bedeutet,
      wobei das Hydroxyalkyl(meth)acrylat der allgemeinen Formel (II) insbesondere ausgewählt ist aus der Gruppe bestehend aus 2-Hydroxyethyl(meth)acrylat, 2-Hydroxypropyl(meth)acrylat oder Hydroxybutyl(meth)acrylat; und
   ac) gegebenenfalls weiteren Comonomeren, die mit den (Meth)acrylaten der allgemeinen Formeln (I) und (II) copolymerisierbar sind, als Komponente C, bevorzugt ausgewählt aus der Gruppe bestehend aus Styrol, Acrylnitril, Vinylacetat, Vinylpropionat, Vinylchlorid, Vinylidenchlorid, Butadien und Additionsprodukten aus Versaticsäureglycidresten und ungesättigten Säuren, insbesondere (Meth)acrylsäure, und
   ad) gegebenenfalls Hilfsmonomeren als Komponente D bevorzugt ausgewählt aus der Gruppe bestehend aus (Meth)acrylsäure, Itaconsäure, Maleinsäure, Fumarsäure, Crotonsäure und den Amiden der genannten Säuren;
   und
b) Um- oder Veresterung des hydroxyfunktionelle Seitenketten aufweisenden Poly(meth)acrylats mit einem (Meth)acrylat oder (Meth)acrylsäure, bevorzugt mit Methyl-, Ethyl-, 2-Ethylhexyl- oder Butyl(meth)acrylat, gegebenenfalls in Gegenwart von Stabilisatoren ausgewählt aus der Gruppe bestehend aus 2,6-Dibutylphenolen wie Di-tert.-butylphenol, p-Kresol, Hydrochinon, Dimethylhydrochinon, Phenothiazinen und Phosphorigsäureestern, in Anwesenheit eines die Um-oder Veresterung katalysierenden Enzyms.

Als Abkürzung für "Methacrylsäure oder Acrylsäure" wird "(Meth)acrylsäure" verwendet, entsprechend wird als Abkürzung für "Methacrylat oder Acrylat" "(Meth)acrylat" verwendet.

Mit Hilfe des erfindungsgemäßen Verfahrens ist die Einführung von funktionellen Gruppen, insbesondere von (Meth)acrylgruppen in Poly(meth)acrylate auf schonende Weise möglich, ohne dass mit einer Spaltung der Estergruppen des Poly(meth)acrylats zu rechnen ist. Des weiteren ist die Herstellung der funktionalisierten Polymere ausgehend von hydroxyfunktionelle Seitenketten aufweisenden Poly(meth)acrylaten möglich, die wesentlich preiswerter sind, als bisher im Stand der Technik eingesetzte mit Glycidylgruppen funktionalisierte Poly(meth)acrylate.

### Schritt a)

In einer bevorzugten Ausführungsform des erfindungsgemäßen Verfahrens werden in Schritt a)
- 10 bis 80 Gew.-%, bevorzugt 20 bis 80 Gew.-%, besonders bevorzugt 30 bis 70 Gew.-% der Komponente A, und
- 10 bis 80 Gew.-%, bevorzugt 20 bis 70 Gew.-%, besonders bevorzugt 20 bis 60 Gew.-% der Komponente B, und
- 0 bis 50 Gew.-%, bevorzugt 0 bis 40 Gew.-%, besonders bevorzugt 5 bis 25 Gew.-% der Komponente C, und
- 0 bis 15 Gew.-%, bevorzugt 0 bis 10 Gew.-%, besonders bevorzugt 0,5 bis 5 Gew.-% der Komponente D eingesetzt.

Die erfindungsgemäß eingesetzten hydroxyfunktionelle Seitenketten aufweisenden Poly(meth)acrylate können nach verschiedenen, dem Fachmann bekannten Verfahren hergestellt werden. Dabei ist die Herstellung durch radikalische Polymerisation bevorzugt.

Die Polymerisation erfolgt im Allgemeinen durch Emulsions-, Lösungs- oder Substanzpolymerisation, wobei die Emulsionspolymerisation oder die Lösungspolymerisation bevorzugt ist.

In einer Ausführungsform werden die hydroxyfunktionelle Seitenketten aufweisenden Poly(meth)acrylate durch Emulsionspolymerisation hergestellt. Bei der Emulsionspolymerisation werden die Komponenten A, B sowie gegebenenfalls C und gegebenenfalls D in Anwesenheit von Wasser, Emulgatoren, Initiatoren und gegebenenfalls Reglern miteinander umgesetzt.

Als Emulgatoren werden im Allgemeinen anionische, nichtionische, kationische oder amphotere Emulgatoren eingesetzt, wobei anionische oder nichtionische Emulgatoren bevorzugt sind. Geeignete anionische Emulgatoren sind Natrium-, Kalium- oder Ammoniumsalze langkettiger aliphatischer Carbonsäuren und Sulfonsäuren, Alkali-C₁₂₋₁₆-Alkylsulfate, oxethylierte und sulfatierte oder sulfonierte langkettige aliphatische Alkohole oder Alkylphenole und Sulfodicarbonsäureester. Geeignete nichtionische Emulgatoren sind oxethylierte Fettalkohole und Alkylphenole, wobei die Ethylenoxideinheiten zwischen 2 und 50 mol/mol betragen können. Geeignete kationische Emulgatoren sind Ammonium-, Phosphonium- und Sulfoniumverbindungen, die als hydrophoben Molekülteil mindestens eine lange aliphatische Kohlenwasserstoftlcette enthalten. Es ist auch möglich, eine Kombination von verschiedenen Emulgatoren, zum Beispiel von ionischen und nichtionischen Emulgatoren, einzusetzen.

Das eingesetzte Wasser ist bevorzugt destilliert oder entsalzt, da Salze die Emulsionsstabilität beeinträchtigen können. Im Allgemeinen wird das Polymerisationsverfahren unter Stickstoff durchgeführt, da Sauerstoff die Polymerisation inhibiert.

Das Molekulargewicht der hydroxyfunktionelle Seitenketten aufweisenden Poly(meth)acrylate kann durch Zugabe von Reglern erniedrigt werden. Geeignete Regler sind zum Beispiel halogenhaltige Verbindungen wie Tetrachlorkohlenstoff, Tetrabromkohlenstoff, Bromal, Benzylbromid und Trichlorbrommethan oder Mercaptane wie Butylinercaptan oder Dodecylmercaptan oder Rongalit ® C.

Als Initiatoren sind im Allgemeinen alle dem Fachmann bekannten Initiatoren zur Polymerisation von (Meth)acrylaten geeignet. Im Allgemeinen werden wasserlösliche Peroxoverbindungen wie Alkali- oder Ammoniumpersulfat, Wasserstoffperoxid oder tert.Butyl-Peroxy-Ethylhexanoat eingesetzt. Des weiteren sind Redox-Systeme wie H₂O₂-Ascorbinsäure, H₂O₂-Fe(II)/Fe(III), H₂O₂-Ce(IV), Persulfite-Fe, Metabisulfite-Fe oder Hydroperoxide-Metallsalze geeignet. Die Initiatoren werden im Allgemeinen in einer Menge von 0,05 bis 8 Gew.-%, bevorzugt 0,2 bis 2 Gew.-%, bezogen auf Menge der eingesetzten Monomere, eingesetzt.

Gegebenenfalls nach der Polymerisation noch vorhandener Initiator kann nach der Polymerisation deaktiviert werden, um eine mögliche Polymerisation der in Schritt b) erfindungsgemäß hergestellten Poly(meth)acrylate zu vermeiden. Die Deaktivierung erfolgt im Allgemeinen durch Zugabe eines Reduktionsmittels, z.B. Ascorbinsäure.

Die Polymerisation wird im Allgemeinen in einem Temperaturbereich von 30 bis 120°C, bevorzugt 40 bis 110°C, besonders bevorzugt 50 bis 90°C durchgeführt. Die Polymerisation wird im Allgemeinen bei einem Druck von 1 bis 20, bevorzugt 1 bis 15 bar, besonders bevorzugt 1 bis 5 bar durchgeführt.

Die Emulgatoren werden im Allgemeinen in einer Menge von 0,5 bis 15 Gew.-%, bevorzugt von 0,5 bis 10 Gew.-%, besonders bevorzugt 0,5 bis 5 Gew.-%, bezogen auf die Menge der eingesetzten Komponenten A, B, gegebenenfalls C und gegebenenfalls D, eingesetzt.

Der Teilchendurchmesser der nach Polymerisation erhaltenen hydroxyfunktionelle Seitenketten aufweisenden Poly(meth)acrylate beträgt im Allgemeinen 20 bis 1000 nm, bevorzugt 20 bis 500 nm, besonders bevorzugt 50 bis 400 nm, ermittelt mittels Lichtstreuung.

Der pH-Wert beträgt bei der Emulsionspolymerisation im Allgemeinen zwischen 1 und 6, bevorzugt zwischen 2 und 6. Die Hydroxylzahlen betragen im Allgemeinen mindestens 20 bis 180, bevorzugt mindestens 40 bis 120. Der Fetsstoffgehalt der Dispersionen beträgt im Allgemeinen 10 bis 50, bevorzugt 20 bis 40 und die Glasübergangstemperatur der erhaltenen Polymere beträgt im Allgemeinen zwischen -40 und +80°C.

Die erhaltenen hydroxyfunktionelle Seitenketten aufweisenden Poly(meth)acrylate weisen im Allgemeinen ein mittleres Molekulargewicht von 1000 bis 2 000 000, bevorzugt 1000 bis 1000 000, besonders bevorzugt 50 000 bis 500 000 auf. Das mittlere Molekulargewicht wurde mittels Gelpermeationschromatographie (GPC) ermittelt. Es handelt sich hierbei um das zahlenmittlere Molekulargewicht.

Die Herstellung der hydroxyfunktionelle Seitenketten aufweisenen Poly(meth)acrylate ist mittels Eintopf- oder Batchfahrweise, Zulaufverfahren und kontinuierlichen Fahrweisen möglich. Die Durchführung der genannten Fahrweisen ist dem Fachmann bekannt.

Das in Schritt a) erhaltene hydroxyfunktionelle Seitenketten aufweisende Poly(meth)acrylat kann nach dem Fachmann bekannten Methoden isoliert werden. Eine Ausführungsweise ist z.B. in EP-A 0 029 637 beschrieben, wobei jedoch in dem Verfahren gemäß der vorliegenden Anmeldung hydroxylgruppenfreie Lösungsmittel eingesetzt werden, beziehungsweise in einem zweiten Schritt ein hydroxylgruppenhaltiges Lösungsmittel durch ein hydroxylgruppenfreies Lösungsmittel ersetzt wird. Für den Einsatz in Schritt b) des erfindungsgemäßen Verfahrens wird das hydroxyfunktionelle Seitenketten aufweisende Poly(meth)acrylat nach Isolierung in wasserfreier Form eingesetzt.

In einer weiteren bevorzugten Ausführungsform werden die hydroxyfunktionelle Seitenketten aufweisenden Poly(meth)acrylate durch Lösungspolymerisation hergestellt. Bei der Lösungspolymerisation werden die Komponenten A, B sowie gegebenenfalls C und gegebenenfalls D in Anwesenheit von Lösungsmittel, Initiator und gegebenenfalls Reglern miteinander umgesetzt.

Für die Lösungspolymerisation geeignete Initiatoren sind Peroxide wie Dialkylperoxide, z.B. Di-tert.-Butylperoxid und Di-tert.-Amylperoxid, Peroxiester wie tert.-Butylperoxy-2-ethylhexanoat und tert.-Amylperoxy-2-ethylhexanoat, Diacylperoxide wie Benzoylperoxid, Lauroylperoxid und Decanoylperoxid, Percarbonate wie tert.-Butylperoxyisopropylcarbonat, Di-2-ethylhexylperoxidicarbonat, Perketale und Ketonperoxide, sowie Azoinitiatoren wie 2,2'-Azobis-(2,4-Dimethyl-pentannitril), 2,2'-Azobis-(2-Methylpropanonitril), 2,2'-Azobis-(2-Methylbutanonitril), 1,1'-Azobis-(Cyclohexancarbonitril), 2,2'-Azobis-(2,4,4-trimethylpentan) und 2-Phenylazo-2,4-dimethyl-4-methoxyvaleronitril.

Bevorzugt sind Lösungsmittel, die für eine enzymatische Umsetzung gemäß Schritt b) nicht störend sind, so dass eine Entfernung des Lösungsmittels vor der Ausführung von Schritt b) nicht erforderlich ist. Besonders bevorzugt werden Lösungsmittel ausgewählt aus Methylisobutylketon, Aceton, Xylol, N-Methylpyrrolidon, Methylethylketon, Methylpropylketon, Methylamylketon und Solventnaphtha, eingesetzt.

### Schritt b)

In Schritt b) erfolgt die Um- oder Veresterung des hydroxyfunktionelle Seitenketten aufweisenden Poly(meth)acrylats mit mindestens einem (Meth)acrylat oder (Meth)acrylsäure oder einem Stabilisator in Anwesenheit eines die Um- oder Veresterung katalysierenden Enzyms. Bevorzugt wird eine Umesterung mit Methyl-, Ethyl-, 2-Ethylhexyl- oder Butyl(meth)acrylat durchgeführt.

Die enzymatische Um- oder Veresterung mit einem (Meth)acrylat oder (Meth)acrylsäure erfolgt im Allgemeinen bei niedrigen Temperaturen, bevorzugt 10 bis 100°C, besonders bevorzugt 20 bis 80°C. Die Reaktionsbedingungen bei der enzymatischen Um- oder Veresterung sind mild. Aufgrund der niedrigen Temperaturen und sonstigen milden Bedingungen wird die Bildung von Nebenprodukten in Schritt b) vermieden, die andernfalls zum Beispiel von chemischen Katalysatoren stammen können oder durch unerwünschte radikalische Polymerisation des eingesetzten (Meth)acrylats oder der eingesetzten (Meth)acrylsäure, die sonst nur durch Zugabe von Stabilisatoren verhindert werden kann.

Zur enzymatischen Umsetzung (Schritt b)) kann das Produkt aus Schritt a) im Allgemeinen ohne weitere Vorbehandlung eingesetzt werden. Bei Bedarf kann das Produkt von flüchtigen Stoffen (z. B. Lösungsmittel) befreit werden oder es können zusätzliche Stoffe (z.B. Lösungsmittel) zugesetzt werden. Speziell sollte es möglichst frei von Radikalstartern sein oder arm an Radikalstartern sein.

Bevorzugt werden als Enzyme Hydrolasen eingesetzt, insbesondere Hydrolasen ausgewählt aus der Gruppe bestehend aus Lipasen, Esterasen und Proteasen. Die Enzyme können in freier Form oder in immobilisierter Form auf einem Träger, auf dem sie chemisch oder physikalisch gebunden sind eingesetzt werden. Die Menge des Enzymkatalysators beträgt bevorzugt 0,1 bis 20 Gew.-%, besonders bevorzugt 1 bis 10 Gew.-%, bezogen auf das eingesetzte hydroxyfunktionelle Seitenketten aufweisende Poly(meth)acrylat.

Die Reaktionszeit hängt unter anderem von der verwendeten Menge und der Aktivität des Enzymkatalysators und dem gewünschten Umsatzgrad ab sowie von der hydroxyfunktionellen Seitenkette des Poly(meth)acrylats.

Das zur Umesterung eingesetzte (Meth)acrylat bzw. die zur Veresterung eingesetzte (Meth)acrylsäure wird im Allgemeinen in äquimolaren Mengen oder einem Überschuss im Verhältnis zur Anzahl der hydroxyfunktionellen Seitenketten im Poly(meth)acrylat eingesetzt. Bevorzugt wird ein Molverhältnis (Meth)acrylat bzw. (Meth)acrylsäure zu Hydroxygruppen in den Seitenketten des Poly(meth)acrylats von 1 : 1 bis 10 : 2 eingesetzt. Höhere Überschüsse sind nicht störend.

Im Allgemeinen werden in Schritt b) 20 - 100 %, bevorzugt 40 bis 100 %, besonders bevorzugt 60 bis 100 % aller ursprünglich im Poly(meth)acrylat enthaltenden hydroxyfunktionellen Seitenketten mit einem (Meth)acrylat oder (Meth)acrylsäure umgesetzt.

Gegebenenfalls eingesetzte geeignete Stabilisatoren sind ausgewählt aus der Gruppe bestehend aus 2,6-Dibutylphenolen wie Di-tert.-butylphenol, p-Kresol, Hydrochinon, Dimethylhydrochinon, Phenothiazinen und Phosphorigsäureestern. Es ist jedoch auch möglich, Schritt b) ohne Einsatz von Stabilisatoren durchzuführen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor, einem Festbettreaktor oder einem Taylorreaktor.

Das während der Um- oder Veresterung entstehende Reaktionswasser bzw. der entstehende Alkohol kann nach dem Fachmann bekannten Methoden, zum Beispiel durch Absorption (zum Beispiel mit Molekularsieb), Destillation oder Pervaporation entfernt werden.

Die Reaktion wird solange durchgeführt, bis der gewünschte Umsatz, von im Allgemeinen 5 bis 100 % erreicht ist. Dabei lassen sich bei einer Reaktionsführung mit gleichzeitiger Entfernung des während der Umsetzung entstehenden Alkohols bzw. Wassers höhere Umsätze in kürzeren Reaktionszeiten aufgrund der Verschiebung des Reaktionsgleichgewichts erreichen.

Der Enzymkatalysator kann im Anschluss an die Umsetzung durch geeignete Maßnahmen, zum Beispiel Filtrieren oder Dekantieren abgetrennt werden und gegebenenfalls mehrfach eingesetzt werden.

Ein weiterer Gegenstand der vorliegenden Anmeldung sind UV- und/oder Dual-Cure-härtbare Poly(meth)acrylate herstellbar nach dem erfindungsgemäßen Verfahren. Aufgrund der milden Reaktionsbedingungen in dem erfindungsgemäßen Verfahren sind neuartige mit (Meth)acryl-funktionelle Poly(meth)acrylate zugänglich, ohne dass die Gefahr einer Spaltung der Esterbindungen in den Poly(meth)acrylaten durch Säurekatalyse oder hohe Temperaturen auftritt.

Diese erfindungsgemäßen (Meth)acryl-funktionellen Poly(meth)acrylate sind als Bindemittel in Strahlen- oder Dual-Cure-härtbaren Beschichtungsmitteln, zum Beispiel in Deckbeschichtungen wie transparenten Klarlacken, aber auch in Grundlacken, Grundierungen und Füllern, geeignet. Die (Meth)acryl-funktionellen Poly(meth)acrylate weisen ausgezeichnete Bewitterungsstabilitäten auf. In Verbindung mit einer Härtungstechnologie (Strahlenhärtung oder Dual-Cure-Härtung) lassen sich weitere Vorteile, zum Beispiel eine Erhöhung der Kratzfestigkeit einer Beschichtung, erzielen. Besonders entscheidend ist jedoch die Verbesserung in der Anwendung durch Einsatz der erfindungsgemäßen (Meth)acrylfunktionellen Poly(meth)acrylate, da sie eine schnelle Trocknung ermöglichen.

Ein weiterer Gegenstand der vorliegenden Anmeldung ist daher die Verwendung der erfindungsgemäßen oder nach dem erfindungsgemäßen Verfahren hergestellten (Meth)acrylfunktionellen Poly(meth)acrylate als Bindemittel in Strahlen oder Dual-Cure-härtbaren Beschichtungsmitteln, bevorzugt in Deckbeschichtungen, besonders bevorzugt in transparenten Klarlacken.

Unter "Dual-Cure" ist zu verstehen, dass die Materialien thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Ein weiterer Gegenstand der vorliegenden Anmeldung sind Lackformulierungen, enthaltend die erfindungsgemäßen oder nach dem erfindungsgemäßen Verfahren herstellbaren (Meth)acryl-funktionellen Poly(meth)acrylate. Dabei können die (Meth)acryl-funktionellen Poly(meth)acrylate oder die mit Stabilisatoren funktionalisierten Poly(meth)acrylate sowohl in Basislacken als auch in Decklacken eingesetzt werden. Aufgrund ihrer besonderen Eigenschaften wie der Erhöhung der Kratzfestigkeit verbunden mit hoher UV-Stabilität einer Beschichtung, ist ihr Einsatz in Deckbeschichtungen bevorzugt.

Im Allgemeinen wird die Zusammensetzung der Deckbeschichtung so ausgewählt, dass der ausgehärtete Decklack im gummielastischen Bereich ein Speichermodul E' von mindestens 10^{7,6} Pa, bevorzugt von mindestens 10⁸'⁰ Pa, besonders bevorzugt von mindestens 10^{8,3} Pa, und einen Verlustfaktor bei 20°C von maximal 1,10, bevorzugt maximal 0,06, aufweist, wobei das Speichermodul E' und der Verlustfaktor tanδ mit der dynamischmechanischen Thermo-Analyse an homogenen freien Filmen mit einer Schichtdicke von 40 ± 10 µm gemessen worden sind. Der Verlustfaktor tanδ ist dabei definiert als der Quotient aus dem Verlustmodul E" und dem Speichermodul E'.

Die dynamisch-mechanische Thermo-Analyse ist eine allgemeine Messmethode zur Bestimmung der viskoelastischen Eigenschaften von Beschichtungen und beispielsweise beschrieben in Murayama T., Dynamic Mechanical Analyse of Polymeric Material, Elsevier, New York, 1978 und Loren W. Hill, Journal of Coatings Technology, Vol. 64, No. 808, May 1992, S. 31 bis 33. Die Durchführung der Messungen kann beispielsweise mit den Geräten II, MKBI, oder MKIV der Firma Rheometrics Scientific erfolgen.

Bevorzugt weisen die Strahlen- oder Dual-Cure-härtbaren Deckbeschichtungen eine Viskosität bei 23°C von < als 100 s Auslaufzeit im DIN4-Becher, besonders bevorzugt < 80 s Auslaufzeit im DIN4-Becher auf. Für Gieß- und Walzenapplikation kann die Viskosität auch darüber liegen.

Die erfindungsgemäßen Deckbeschichtungen enthalten neben den erfindungsgemäßen (Meth)acryl-fnnktionellen Poly(meth)acrylaten gegebenenfalls einen oder mehrere Photoinitiatoren sowie gegebenenfalls übliche Hilfs- und Zusatzstoffe. Geeignete Photoinitiatoren sind übliche, in Strahlen- oder Dual-Cure-härtbaren Beschichtungsmitteln eingesetzte Photoinitiatoren, beispielsweise Benzophenone, Benzoine oder Benzoinether, bevorzugt Hydroxyacrylketone und Bis(acyl)phosphinoxide. Es können auch beispielsweise die im Handel unter den Namen Irgacure® 184, Irgacure® 1800 und Irgacure® 500 der Firma Ciba Geigy, Genocure® MBF der Firma Rahn und Lucirin® TPO der Firma BASF AG eingesetzt werden.

Als weitere Hilfs- und Zusatzstoffe sind beispielsweise Lichtschutzmittel (zum Beispiel HALS-Verbindungen, Benztriazole, Oxalanilid und andere), Slipadditive, Polymerisationsinhibitoren, Mattierungsmittel, Entschäumer, Verlaufsmittel und filmbildende Hilfsmittel, zum Beispiel Zellulose-Derivate und andere, geeignet. Außerdem können rheologiesteuernde Komponenten eingesetzt werden, wie organische Harnstoffverbindungen, Urethanharnstoffverbindungen und/oder SiO₂.

Die erfindungsgemäßen Deckbeschichtungen kommen insbesondere als Klarlacke zum Einsatz, so dass sie üblicherweise keine oder nur transparente Füllstoffe und keine deckenden Pigmente enthalten. Es ist aber auch der Einsatz in Form von pigmentierten Deckbeschichtungen möglich. In diesem Fall enthalten die Deckbeschichtungen zusätzlich Pigmente. Ferner können die Deckbeschichtungen in diesem Fall einen oder mehrere Füllstoffe enthalten.

Ein weiterer Gegenstand der vorliegenden Anmeldung sind somit Deckbeschichtungen, enthaltend
5 bis 80 Gew.-%, bevorzugt 10 bis 60 Gew.-%, besonders bevorzugt 20 bis 50 Gew.-% mindestens eines erfindungsgemäßen oder nach dem erfindungsgemäßen Verfahren hergestellten (Meth)acryl-funktionellen Poly(meth)acrylats,
0,5 bis 15 Gew.-%, bevorzugt 1 bis 10 Gew.-%, besonders bevorzugt 1 bis 5 Gew.-% mindestens eines Photoinitiators,
0,5 bis 8 Gew.-%, bevorzugt 1 bis 6 Gew.-%, besonders bevorzugt 1 bis 4 Gew.-% weiterer Hilfs- und Zusatzstoffe,
0 bis 40 Gew.-%, bevorzugt 0 bis 30 Gew.-%, besonders bevorzugt 0 bis 25 Gew.-% Pigmente,
und 0 bis 40 Gew.-%, bevorzugt 0 bis 30 Gew.-%, besonders bevorzugt 0 bis 25 Gew.-% mindestens eines Füllstoffs, wie transparente Metalloxide, BaSO₄ und Wachse.

Bevorzugte (Meth)acryl-funktionelle Poly(meth)acrylate, Photoinitiatoren, Hilfs- und Zusatzstoffe und Füllstoffe und Pigmente sind bereits vorstehend genannt.

Die Herstellung der erfindungsgemäßen Deckbeschichtungen erfolgt durch Mischen der einzelnen Komponenten gemäß dem Fachmann bekannten Verfahren in dem Fachmann bekannten Vorrichtungen.

Ein weiterer Gegenstand der vorliegenden Anmeldung ist daher ein Verfahren zur Herstellung der erfindungsgemäßen Deckbeschichtung, worin das (Meth)acryl-funktionelle Poly(meth)acrylat, der Photoinitiator, ggf. weitere Hilfs- und Zusatzstoffe und ggf. Füllstoffe und Pigmente miteinander gemischt werden.

Die erfindungsgemäßen Deckbeschichtungen werden im Allgemeinen auf mit einem Basislack beschichtete Substrate aufgetragen. Sie können durch sogenanntes coil coating oder durch Spritzguß auf die Substrate aufgebracht werden. Solche Substrate sind beispielsweise Metallbleche, bzw. Metallbänder und Kunststoffe jeder Art, z.B. Automobilkarosserien und Motorradteile..

Nach Auftragen der Deckbeschichtung wird diese einer Strahlen- oder Dual-Cure-Härtung unterworfen. Die Anlagen und Bedingungen für diese Härtungsmethoden sind aus der Literatur bekannt und bedürfen keiner weiteren Beschreibung (für Strahlungshärtung siehe zum Beispiel R. Holmers, UV and E. B. Curing Formulations for Printing Inks, Coatings and Paints, SITA Technology, Academic Press, London, United Kingdom 1984).

Die nachfolgenden Beispiele erläutern die Erfindung zusätzlich.

### Beispiele

### 1. Herstellung eines hydroxyfunktionellen Bindemittels

### Rezeptur:

| | | | |
|---|---|---|---|
| **Vorlage** | Methylisobutylketon | | 540,0 g |
| **Monomere** | Styrol | 10,00 Gew.-% ¹⁾ | 123,6 g |
| | EHA (2-Ethylhexylacrylat) | 46,50 Gew.-% ¹⁾ | 574,4 g |
| | HEMA (Hexylethylmethacrylat) | 27,00 Gew.-% ¹⁾ | 336,6 g |
| | HBA (Hydroxybutylacrylat) | 15,00 Gew.-% ¹⁾ | 185,2 g |
| | AS (Acrylsäure) | 1,50 Gew.-% ¹⁾ | 18,6 g |
| spülen | Methylisobutylketon | | 5,0 g |
| **Initiator** | tert.-Butyl-peroxy-2-Ethylhexanoat | 8 Gew.-% ²⁾ | 98,8 g |
| | Methylisobutylketon | | 74,2 g |
| spülen | Methylisobutylketon | | 46,6 g |
| **End** | | | 2.000,0 g |

| | | | |
|---|---|---|---|
| 1) bezogen auf die Summe der Komponenten Styrol, EHA, HEMA, HBA, AS 2) bezogen auf die Summe der Komponenten Styrol, EHA, HEMA, HBA, AS | | | |

### Verfahren:

Vorlage einwiegen und auf 110°C aufheizen. Bei konstanter Temperatur Monomere und Initiator gleichmäßig in den Reaktor zudosieren. Nach 4 Stunden ist der Monomerenzulauf beendet. Nach 4,5 Stunden ist der Initiatorzulauf beendet. Nach dem Ende der Initiatordosierung wird 1 Stunde nachpolymerisiert und anschließend abgekühlt und die erhaltene Reaktionsmischung abgelassen.

***Endwerte:**

| | |
|---|---|
| Festkörper (1h, 130°C): | 66,3 % |
| OH-Zahl (theor.) gesamt: | 174,8 mg/g (ermittelt nach DIN 43402) |
| OH-Zahl (praktisch): | 165 mg/g (ermittelt nach DIN 53246) |
| GC (Restgehalt Monomere)³⁾: | EHA 0,3 %; AS < 0,3 % alle anderen < 0,1 % |
| GPC⁴⁾ Mn⁵⁾ | 5829 |
| M_{w}⁶⁾ | 20722 |
| M_{w}/Mₙ⁷) | 3,55 |

| | |
|---|---|
| 3) GC = Gaschromatographie 4) GPC = Gelpermeationschromatographie (mit Polystyrostandard) 5) Mₙ = zahlenmittleres Molekulargewicht 6) M_{w} = gewichtsmittleres Molekulargewicht 7) M_{w}/Mₙ= Polydispersität | |

### 2. Herstellung eines UV-aktiven Polyacrylats

| | |
|---|---|
| Ansatz: | 300 ml Polymerlösung in Methylisobutylketon aus Beispiel 1 |
| | 300 g Methylacrylat (MA) |
| | 150 mg Methoxyphenol |
| | 150 g Molsieb 5 Å |
| | 30 g Novozym® 435 (immobilisierte Lipase aus Candida antarctica der Firma Novozymes) |

Die genannten Komponenten werden 72 Stunden bei 40°C gerührt. Anschließend wird die Reaktionsmischung filtriert und das erhaltene Polyacrylat mit Methylisobutylketon (MIK) nachgewaschen. Der Überschuss MA und MIK wird bei 60°C bis 70°C am Rotationsverdampfer im Vakuum entfernt. Man erhielt 227 g Wertprodukt. Der Anteil der acrylierten Hydroxygruppen wurde mittels OH-Zahl zu etwa 34 % bestimmt.

Die Ermittlung der OH-Zahl erfolgte nach einer im Stand der Technik bekannten Methode (DIN 53240, Teil 2).

### 3. Herstellung einer UV-Lackformulierung

### a) Stammlack

| | |
|---|---|
| erfindungsgemäßes UV-Polyacrylat | 32,5 |
| Sartomer® 399 | 30,6 |
| Thixharz® SCA | 11,5 |
| (Basis: Benzylamin/Hexamethylendiisocyanat) | |
| Irgacure® 184 | 0,8 |
| (Photoinitiator) | |
| Lucirin® TPO | 0,4 |
| (Photoinitiator) | |
| Byk® 358 | 0,2 |
| (Verlaufshilfsmittel) | |
| Tinuvin® 292 | 1,0 |
| (Radikalfänger) | |
| Tinuvin®400 | 1,0 |
| (UV-Absorber) | |
| Butylacetat | 22,0 |

### b) Härtermischung

Es wird eine Härtermischung aus 72,7 Teilen Roskydal® UA VP LS 2337 (ungesättigtes Isophorondiisocyanat), 18,2 Teilen Roskydal® UA VP FWO 3003 77 und 9,1 Teilen Butylacetat zugegeben (bei den Teilen handelt es sich um Gewichtsteile).

Die Komponenten wurden mit einem Dissolver gemischt.

### 4. Die Lackformulierung wurde durch Spritzapplikation appliziert.

### 5. Variation der hydroxyfunktionellen Einheiten

Wie folgende Beispiele zeigen, können verschiedene hydroxyfunktionelle Einheiten eingesetzt werden. Die erwähnten Beispiele sollen aber keine Beschränkung sein. Die Polymerlösungen wurden nach Verfahren entsprechend Beispiel 1 hergestellt. 10 g Polymerlösung, 10 g Methylacrylat, 5 g Molsieb (5 Å) und 1 g immobilisierte Lipase (Novozym→ 435) wurden 72 Stunden bei 40°C geschüttelt. Nach Filtration und Einengen wurde der Umsatz über die OH-Zahl bestimmt.

| Polymerlösung | veresterte Einheit | Umsatz [%] |
|---|---|---|
| 2 | Hydroxyethyl-acrylat | 34 |
| 3 | Hydroxyethyl-acrylat | 41 |
| 4 | Hydroxyethyl-methacrylat | 12 |
| 5 | Hydroxyethyl-methacrylat | 22 |
| 6 | Hydroxyethyl-methacrylat | 47 |
| 7 | Hydroxybutyl-acrylat | 67 |
| 8 | Hydroxybutyl-acrylat | 80 |

### 6. Reaktionsoptimierung

Die Reaktionsbedingungen konnten optimiert werden durch Variation der Reaktionszeit, der zugegebenen Menge Methylacrylat und Molsieb. Die in der Tabelle unter Beispiel 5 gezeigten Polymerlösungen wurden unter folgenden optimierten Reaktionsbedingungen umgesetzt.

10 g Polymerlösung, 2 g Methacrylat, 2 g Molsieb und 1 g Novozym® 435 wurden 24 Stunden bei 40°C geschüttelt. Nach Filtration und Einengen wurde der Umsatz über die OH-Zahl bestimmt.

| Polymerlösung | Umsatz [%] |
|---|---|
| 2 | 23 |
| 3 | 39 |
| 5 | 6 |
| 6 | 17 |
| 7 | 40 |
| 8 | 46 |

## Patentansprüche

1. Verfahren zur Herstellung von mit aktinischer Strahlung und/oder Dual-Cure-härtbarer Poly(meth)acrylate umfassend die folgenden Schritte
a) Herstellung eines hydroxyfunktionelle Seitenketten aufweisenden Poly(meth)acrylats durch Polymerisation von
aa) mindestens einem (Meth)acrylat der allgemeinen Formel (1), als Komponente A 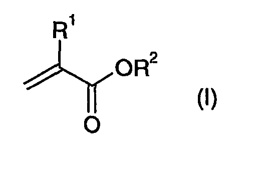 worin
R¹ H, CH₃ oder CH₂OH bedeutet, und
R² ein Alkylrest ist, der gegebenenfalls mit funktionellen Gruppen wie Acryl-, Ether-, Amino-, Epoxy-, Halogen- oder Sulfonsäuregruppen substituiert ist, und
ab) mindestens einem Hydroxyalkyl(meth)acrylat der allgemeinen Formel (II), als Komponente B 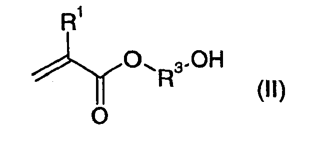 worin
R¹ H, CH₃ oder CH₂OH bedeutet, und
R³ -(CH₂)ₙ-, -CH₂-CH(CH₃)-CH₂- oder -CH₂CH(CH₃)- oder -CH(CH₃)CH₂- oder 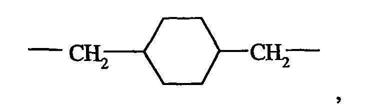
n mindestens 2 bedeutet, und
ac) gegebenenfalls weiteren Comonomeren, die mit den (Meth)acrylaten der allgemeinen Formeln (I) und (II) copolymerisierbar sind, als Komponente C, und
ad) gegebenenfalls Hilfsmonomeren, als Komponente D;
und
b) Um- oder Veresterung des hydroxyfunktionelle Seitenketten aufweisenden Poly(meth)acrylats mit einem (Meth)acrylat oder (Meth)acrylsäure in Anwesenheit eines die Um- oder Veresterung katalysierenden Enzyms.

2. Verfahren nach Anspruch 1, **dadurch gekennzeichnet, dass** in Schritt a)
- 10 bis 80 Gew.-% der Komponente A,
- 10 bis 80 Gew.-% der Komponente B,
- 0 bis 50 Gew.-% der Komponente C und
- 0 bis 15 Gew.-% der Komponente D
eingesetzt werden.

3. Verfahren nach Anspruch 1 oder 2, **dadurch gekennzeichnet, dass** in Schritt b) als Enzyme Hydrolasen ausgewählt aus der Gruppe bestehend aus Lipasen, Esterasen und Proteasen eingesetzt werden.

4. Verfahren nach einem der Ansprüche 1 bis 3, **dadurch gekennzeichnet, dass** in Schritt b) Methyl-, Ethyl-, 2-Ethylhexyl- oder Butyl(meth)acrylat eingesetzt wird.

5. Verfahren nach einem der Ansprüche 1 bis 4, **dadurch gekennzeichnet, dass** Schritt b) bei einer Temperatur von 20 bis 100°C, bevorzugt 20 bis 80°C durchgeführt wird.

6. Verfahren nach einem der Ansprüche 1 bis 5, **dadurch gekennzeichnet, dass** die Komponente B ausgewählt ist aus der Gruppe bestehend aus 2-Hydroxyethyl(meth)acrylat, 2-Hydroxypropyl(meth)acrylat und Hydroxybutyl(meth)acrylat.

7. Verfahren nach einem der Ansprüche 1 bis 6, **dadurch gekennzeichnet dass** 5 bis 100 % der Seitenketten des gemäß Schritt a) hergestellten Poly(meth)acrylats (meth)acryliert sind.

8. Mit aktinischer Strahlung und/oder Dual-Cure-härtbare Poly(meth)acrylate herstellbar nach einem Verfahren gemäß einem der Ansprüche 1 bis 7.

9. Verwendung von mit aktinischer Strahlung und/oder Dual-Cure-härtbaren Poly(meth)acrylaten nach Anspruch 8 oder hergestellt nach einem Verfahren gemäß einem der Ansprüche 1 bis 7 als Komponente bei der Herstellung von Dispersionen oder als Komponente in Lackformulierungen, bevorzugt in mit aktinischer Strahlung und/oder Dual-Cure härtbaren Beschichtungen oder Deckbeschichtungen, besonders bevorzugt in transparenten Klarlacken.

10. Deckbeschichtung enthaltend
- 5 bis 80 Gew.-% mindestens eines mit aktinischer Strahlung und/oder Dual-Cure-härtbaren Poly(meth)acrylats gemäß Anspruch 8 oder hergestellt nach einem der Ansprüche 1 bis 7,
- 0,5 bis 15 Gew.-% mindestens eines Photoinitiators,
0,5 bis 8 Gew.-% weiterer Hilfs- und Zusatzstoffe,
0 bis 40 Gew.-% Pigmente, und
0 bis 40 Gew.-% mindestens eines Füllstoffs.

11. Verfahren zur Herstellung einer Deckbeschichtung gemäß Anspruch 10, worin die einzelnen Komponenten miteinander gemischt werden.

12. Verwendung einer Lackformulierung gemäß Anspruch 10 als Deckbeschichtung.

## Claims

1. A process for preparing poly(meth)acrylates curable with actinic radiation and/or dual-cure poly(meth)acrylates, comprising the following steps:
a) preparing a poly(meth)acrylate containing hydroxy-functional side chains by polymerizing
aa) at least one (meth) acrylate of the general formula (I) as component A 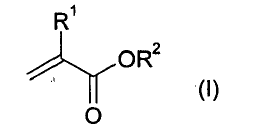 in which
R¹ is H, CH₃ or CH₂OH and
R² is an alkyl radical which is optionally substituted by functional groups such as acrylic, ether, amino, epoxy, halogen or sulfonic acid groups, and
ab) at least one hydroxyalkyl (meth)acrylate of the general formula (II) as component B 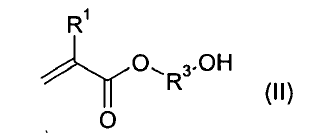 in which
R¹ is H, CH₃ or CH₂OH and
R³ is -(CH₂)ₙ-, -CH₂-CH (CH₃) -CH₂- or -CH₂CH (CH₃) - or -CH (CH₃) CH₂- or 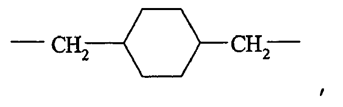
n is at least 2, and
ac) if appropriate, further comonomers, copolymerizable with the (meth)acrylates of the general formulae (I) and (II), as component C, and
ad) if appropriate, auxiliary monomers as component D;
and
b) transesterifying or esterifying the poly(meth)acrylate containing hydroxy-functional side chains with a (meth)acrylate or (meth)acrylic acid in the presence of an enzyme which catalyzes the transesterification or esterification.

2. The process according to claim 1, wherein step a) is carried out using
- 10 to 80% by weight of component A,
- 10 to 80% by weight of component B,
- 0 to 50% by weight of component C, and
- 0 to 15% by weight of component D.

3. The process according to claim 1 or 2, wherein enzymes used in step b) are hydrolases selected from the group consisting of lipases, esterases, and proteases.

4. The process according to any of claims 1 to 3, wherein step b) is carried out using methyl, ethyl, 2-ethylhexyl or butyl (meth)acrylate.

5. The process according to any of claims 1 to 4, wherein the temperature at which step b) is conducted is 20 to 100°C, preferably 20 to 80°C.

6. The process according to any of claims 1 to 5, wherein component B is selected from the group consisting of 2-hydroxyethyl (meth)acrylate, 2-hydroxypropyl (meth)acrylate, and hydroxybutyl (meth)acrylate.

7. The process according to any of claims 1 to 6, wherein 5 to 100% of the side chains of the poly(meth)acrylate prepared in accordance with step a) have been (meth)acrylated.

8. A poly(meth)acrylate curable with actinic radiation and/or dual-cure poly (meth) acrylates preparable by a process according to any of claims 1 to 7.

9. The use of poly (meth) acrylates curable with actinic radiation and/or dual-cure poly(meth)-acrylates according to claim 8 or prepared by a process according to any of claims 1 to 7 as a component in the preparation of dispersions or as a component in coating formulations, preferably in actinic-radiation-curable and/or dual-cure coatings or topcoats, more preferably in transparent clearcoat materials.

10. A topcoat comprising
- 5 to 80% by weight of at least one poly(meth)acrylate curable with actinic radiation and/or dual-cure poly(meth)acrylate according to claim 8 or prepared according to any of claims 1 to 7,
- 0.5 to 15% by weight of at least one photoinitiator,
0.5 to 8% by weight of further auxiliaries and additives,
0 to 40% by weight of pigments, and
0 to 40% by weight of at least one filler.

11. A process for preparing a topcoat according to claim 10, in which the individual components are mixed with one another.

12. The use of a coating formulation according to claim 10 as a topcoat.

## Revendications

1. Procédé de préparation de poly(méth)acrylates durcissables par un rayonnement actinique et/ou à double durcissement (Dual Cure), comprenant les étapes suivantes
a) préparation d'un poly(méth)acrylate présentant des chaînes latérales à fonctionnalité hydroxy par polymérisation de
aa) au moins un (méth)acrylate de formule générale (I), comme composant A 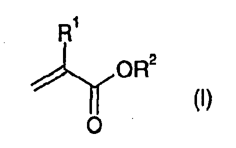 dans laquelle
R¹ signifie H, CH₃ ou CH₂OH, et
R² représente un radical alkyle, qui est le cas échéant substitué par des groupes fonctionnels tels que des groupes acryle, éther, amino, époxy, halogène ou acide sulfonique, et
ab) au moins un (méth)acrylate d'hydroxyalkyle de formule générale (II), comme composant B 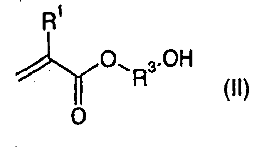 dans laquelle
R¹ signifie H, CH₃ ou CH₂OH, et
R³ signifie - (CH₂)ₙ-, -CH₂-CH (CH₃) -CH₂- ou -CH₂CH (CH₃) - ou -CH(CH₃)CH₂- ou 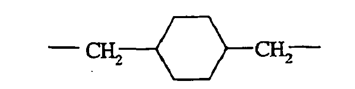 n signifie au moins 2, et
ac) le cas échéant d'autres comonomères, qui sont copolymérisables avec les (méth)acrylates des formules générales (I) et (II), comme composant C, et
ad) les cas échéant des monomères auxiliaires, comme composant D ;
et
b) transestérification ou estérification du poly(méth)acrylate présentant des chaînes latérales à fonctionnalité hydroxy avec un (méth)acrylate ou un acide (méth) acrylique en présence d'une enzyme catalysant la transestérification ou l'estérification.

2. Procédé selon la revendication 1, **caractérisé en ce qu'**on utilise, dans l'étape a)
- 10 à 80% en poids du composant A,
- 10 à 80% en poids du composant B,
- 0 à 50% en poids du composant C et
- 0 à 15% en poids du composant D.

3. Procédé selon la revendication 1 ou 2, **caractérisé en ce que**, dans l'étape b), on utilise comme enzymes des hydrolases choisies dans le groupe formé par les lipases, les estérases et les protéases.

4. Procédé selon l'une quelconque des revendications 1 à 3, **caractérisé en ce qu'**on utilise, dans l'étape b), du (méth)acrylate de méthyle, d'éthyle, de 2-éthylhexyle ou de butyle.

5. Procédé selon l'une quelconque des revendications 1 à 4, **caractérisé en ce que** l'étape b) est réalisée à une température de 20 à 100°C, de préférence de 20 à 80°C.

6. Procédé selon l'une quelconque des revendications 1 à 5, **caractérisé en ce que** le composant B est choisi dans le groupe constitué par le (méth)acrylate de 2-hydroxyéthyle, le (méth)acrylate de 2-hydroxypropyle et le (méth)acrylate d'hydroxybutyle.

7. Procédé selon l'une quelconque des revendications 1 à 6, **caractérisé en ce que** 5 à 100% des chaînes latérales du poly(méth)acrylate préparé selon l'étape a) sont (méth)acrylés.

8. Poly(méth)acrylates durcissables par un rayonnement actinique et/ou à double durcissement, pouvant être préparés selon un procédé selon l'une quelconque des revendications 1 à 7.

9. Utilisation de poly(méth)acrylates durcissables par un rayonnement actinique et/ou à double durcissement selon la revendication 8 ou préparés selon un procédé selon l'une quelconque des revendications 1 à 7 comme composant lors de la préparation de dispersions ou comme composant dans des formulations de laque, de préférence dans des revêtements ou des recouvrements durcissables par un rayonnement actinique et/ou à double durcissement, de manière particulièrement préférée dans les laques claires transparentes.

10. Recouvrement contenant
- 5 à 80% en poids d'au moins un poly(méth)acrylate durcissable par un rayonnement actinique et/ou à double durcissement selon la revendication 8 ou préparé selon l'une quelconque des revendications 1 à 7,
- 0,5 à 15% en poids d'au moins un photo-initiateur,
- 0,5 à 8% en poids d'autres adjuvants et additifs,
- 0 à 40% en poids de pigments, et
- 0 à 40% en poids d'au moins une charge.

11. Procédé pour la préparation d'un recouvrement selon la revendication 10, dans lequel les différents composants sont mélangés les uns aux autres.

12. Utilisation d'une formulation de laque selon la revendication 10 comme recouvrement.
